# EUROPEAN PATENT APPLICATION

(11) **EP 0 692 259 A2**
(43) Date of publication of application: **17.01.1996**
(21) Application number: 95201561.8
(22) Date of filing: 13.06.1995
(51) Int. Cl.: A61K 39/395

(54) **Use of HCG-specific antibody for the treatment of brain neoplasms**

(30) Priority: 13.06.1994 US 258860
(71) Applicant: Allegheny Health Education and Research Foundation, Pittsburgh, Pennsylvania 15222-3009 (DE); InVitro Technologies Inc., Ithaca, New York 14850 (US)
(72) Inventor: Acevedo, Hernan, Pittsburgh, Pennsylvania 15237 (US); Krichevsky, Alexander, Pittsburgh, Pennsylvania 15212 (US); Leavitt, Marc, Pittsburgh, Pennsylvania 15237 (US)
(74) Representative: Van Someren, Petronella F. H. M.

(57) **Abstract**

A method of treating malignant brain cells, and a pharmaceutical composition therefor, by transporting or injecting into the area of those cells at least one monoclonal antibody which specifically reacts with an epitope present on the carboxy-terminal peptide of the beta (β) subunit of human chorionic gonadotropin. The antibody(ies) may be carried across the blood-brain barrier by stereotactic injection, or may be administered intraarterially (intracarotid administration, for example) in conjunction with an osmotic agent which allows at least some of the monoclonal antibody(ies) to pass the blood-brain barrier. Three examples of this class of monoclonal antibodies--CTP101, CTP102 and CTP103--have been administered in an established animal model to show efficacy of treatment of human brain cancer.

## Description

### Field of the Invention

The present invention is a method for treating brain neoplasm, and a pharmaceutical dosage form for use in that treatment, by administering across the blood-brain barrier one or more antibodies which specifically react with an epitope present on the beta (β) subunit of human chorionic gonadotropin or a beta subunit fragment thereof.

### Background of the Invention

Brain neoplasm is a medical condition for which traditional treatments have been notoriously ineffective. Even so-called benign brain tumors, or "space-occupying lesions," could disable and kill a patient simply from the pressure and distortion brought to bear on the brain. Moreover, brain tumors are a very important health problem for all age groups, and the available data suggest that their incidence is increasing. In infants and young children, tumors of the brain are the second most common form of cancer, while in adolescents and young adults they range from the fifth to the eighth most frequent cancers. According to a recent review, it is estimated that the new cases of primary brain tumors in the U.S. for 1993 will be 17,500 cases, an amount higher than primary cancers of the ovary and of malignant lymphomas. Gliomas represent around 60% of primary brain tumors.

Brain cancers have traditionally proved less responsive to conventional cancer therapies than other cancers, often due to a cancer drug's inability to penetrate the so-called blood-brain barrier. Another circumstance frequently encountered with brain cancer is that the brain tumor is not a primary tumor, but represents metastasis of a primary tumor in the lung, breast, ovary, testicle, etc. A patient with metastasis to the brain thus has often already been physically debilitated as a result of the primary cancer itself, and very likely also the treatment of that primary cancer, and accordingly is in desperate need of a tolerable, effective and targeted treatment to destroy the brain lesion(s) and to leave a healthy brain intact.

Exemplary prior art brain cancer treatments include the following. Traditional radiation treatments have been used with varying degrees of success. Surgical treatment techniques include gamma knife stereotactic radiosurgery, among others. Hyperthermia of brain tumors has been used alone and in conjunction with radiation treatment. On occasion, lytic cells (such as recombinant interleukin-2-stimulated peripheral blood lymphocytes from patients having malignant brain tumors) have been intrathecally injected for adoptive transfer, with good results in some cases. Patients have also been treated with some success with the administration of the pineal hormone melatonin.

An inescapable reality with brain cancer and its prior art treatments, however, is that in no case has the long-range outlook traditionally been good for the patient. Even the most recent improvements in treatment have generally been measured in added months of patient survival, rather than years. When patients are treated with surgery, radiotherapy, chemotherapy and interstitial radiation implantation, either alone or combined, the median survival time of malignant glioma patients remains less than one year despite treatment.

Accordingly, a need remains for a treatment for brain neoplasm which is genuinely safe (low or negligible toxicity, few or negligible side effects), and truly effective against cancer of the brain. Moreover, the need is not for a treatment that will impede brain tumor growth, but for a method of differential cellular attack which drastically reduces or obliterates the brain tumor without harming nearby healthy brain cells.

### Summary of the Invention

The present invention is a method of treating neoplastic brain cells by transporting or injecting into the area of those cells at least one monoclonal antibody which specifically reacts with an epitope present on the carboxy-terminal peptide of the beta (β) subunit of human chorionic gonadotropin (or a beta subunit fragment containing that peptide). The antibody(ies) may be carried across the blood-brain barrier by any available means, including but not limited to stereotactic injection or intraarterial (i.e., intracarotid) administration in conjunction with an osmotic agent which allows at least some of the monoclonal antibody(ies) to pass the blood-brain barrier. Three examples of this class of monoclonal antibodies--CTP101, CTP102 and CTP103--have been administered in an established animal model to show efficacy of treatment of human brain cancer, and thus these three antibody examples establish the brain cancer treatment efficacy of the class of monoclonal antibodies specific to an epitope present on the beta (β) subunit of human chorionic gonadotropin or a beta subunit fragment containing that peptide. The invention also embraces a pharmaceutical composition containing at least one mAb so described, together with a pharmaceutically acceptable vehicle suitable for administration into the cerebrospinal fluid.

### Brief Description of the Figures

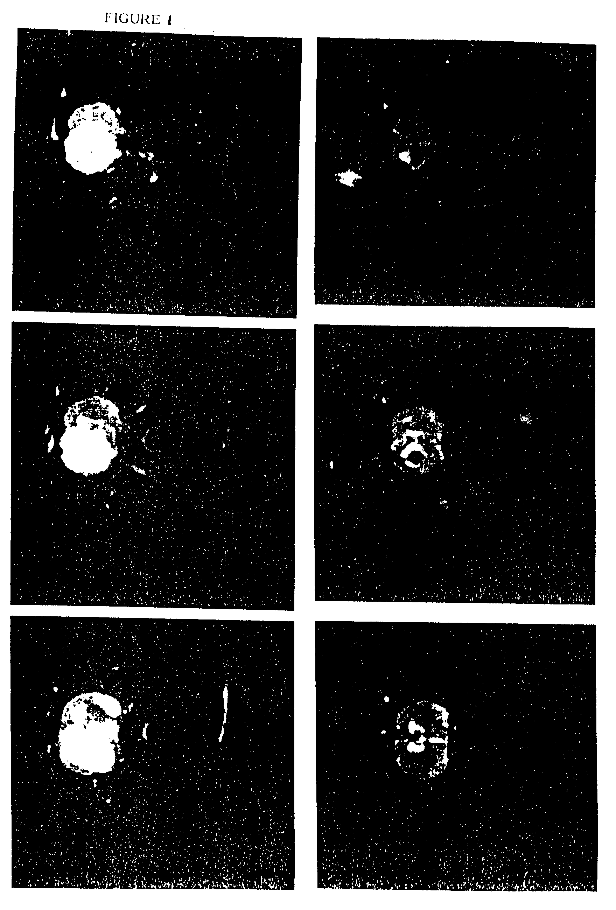

Figure 1 shows adjacent 2 mm thick coronal NMR images through the tumor region of the brain of rat C6-3. Images are arranged left to right from the caudal to the rostral pole of the brain. Pre-treatment images (top row) were obtained at 1 day before starting a 2 week intracerebral hCG-mAb infusion. Post-treatment imaging (bottom row) was performed 15 days later.

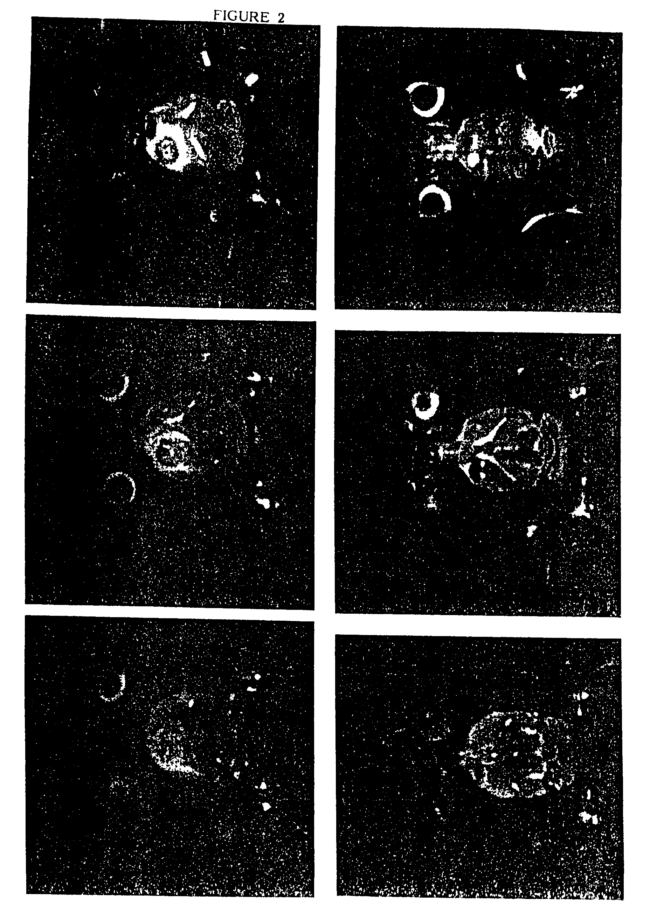

Figure 2 shows adjacent 2 mm thick horizontal NMR images through the tumor region of the brain of rat C6-3. Images are arranged left to right from the inferior to the superior aspect of the brain. Imaging occurred on the seventeenth day post-inoculation (upper row) which was 1 day before the start of mAb treatment and again on day 32 immediately after 2 weeks of intracerebral infusion of mAb (lower row).

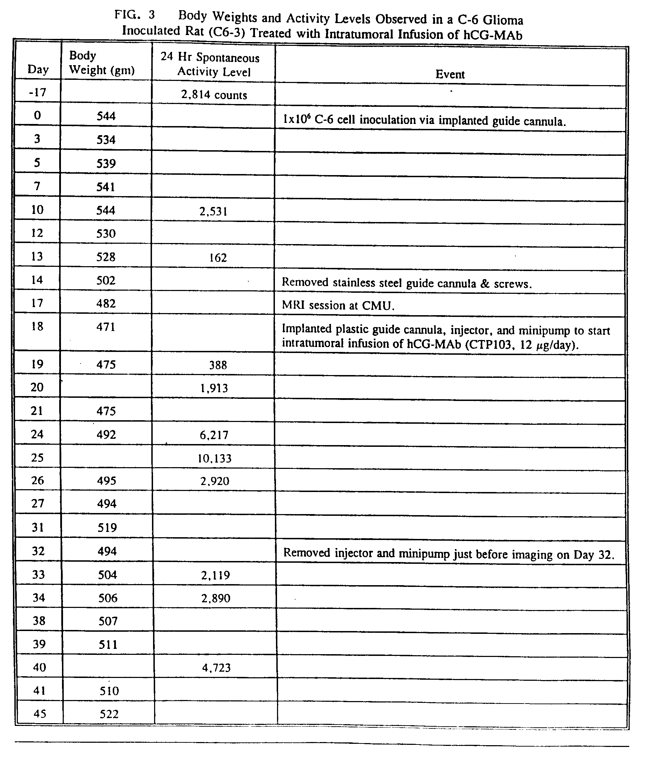

Figure 3 is a table showing the body weights and activity levels observed in a C-6 glioma inoculated rat (C6-3) treated with intratumoral infusion of hCGβ-CTP mAb.

### Detailed Description of the Invention

The present invention is a method of treating malignant brain cells and cancerous brain tumors by injecting into the cell area or tumor at least one monoclonal antibody which specifically reacts with an epitope present on the carboxy terminal peptide of the beta (β) subunit of human chorionic gonadotropin (or a beta subunit fragment containing that peptide). The antibody(ies) may be carried across the blood-brain barrier by stereotactic injection, or may be administered intraarterially (intracarotid administration, for example) in conjunction with an osmotic agent which allows at least some of the monoclonal antibody(ies) to pass the blood-brain barrier. Three examples of this class of monoclonal antibodies--CTP101, CTP102 and CTP103--have been administered to treat brain glioma in a recognized animal model to establish, by correlation, efficacy of treatment of human brain cancer, and thus have likewise established the efficacy of the entire class of monoclonal antibodies specific to an epitope present on the beta (β) subunit of human chorionic gonadotropin (or a beta subunit fragment containing that peptide).

It is already known in a general way to lyse malignant cells with various monoclonal antibodies (mAbs), especially mAbs specific for a wide variety of epitopes present on the β subunit of human chorionic gonadotropin (see for example U.S. Patent No. 4,460,561 to Goldenberg). Human chorionic gonadotropin (hCG) is best known as the hormone of pregnancy, and is a dimeric sialoglycoprotein composed of two noncovalently linked dissimilar subunits known as the α and β subunits. Placental cells, sperm cells and certain fetal tissues synthesize these subunits. In human beings, the single α-subunit (hCGα) gene is located in chromosome 6. A cluster of at least six genes located in chromosome 19 encode individually for the β subunit (hCGβ). The β subunits are important because they determine biological specificity.

Even though a wide variety of mAbs against the β subunit of hCG are known in the art, now for the first time a particular class of these monoclonal antibodies has been identified as possessing a superior and dramatic ability to destroy brain cell malignancies and tumors without affecting healthy brain tissue. This class of monoclonal antibodies specifically reacts with an epitope present on the carboxy-terminal peptide of the beta (β) subunit of human chorionic gonadotropin. The dramatic and superior results have been established in an animal model accepted in the art as predictive of and correlating with comparable results in humans undergoing the same treatment.

For the sake of brevity, a useful abbreviation for "the carboxy-terminal peptide of human chorionic gonadotropin" is "hCGβ-CTP" or sometimes even just "βCTP." For the purposes of the present invention, all monoclonal antibodies specific for hCGβ-CTP (or βCTP) are included within the present method for treating brain cancer, and they may be administered singly or in combination. The terminology "monoclonal antibodies specific for hCGβ-CTP" can be abbreviated "anti-hCGβ-CTP mAbs," or "hCGβ-CTP mAbs," or even just "CTP mAbs," for the purpose of the ensuing disclosure. One or more of these monoclonal antibodies may be administered directly into the brain tissue by stereotactic injection, or administration past the blood-brain barrier may be accomplished with intraarterial administration of the mAb(s) together with an osmotic agent which alters the blood-brain barrier enough to allow some of the mAb(s) to pass through it. Osmotic agents known to alter blood-brain barrier transport are known in the art. Usually the CTP mAb(s) are administered in conjunction with at least one pharmaceutically acceptable vehicle compatible with cerebrospinal fluid.

References to hCGβ-CTP and antibodies which bind it are abundant in the literature. In Birken, S., et al., "Characterization of Antisera Distinguishing Carbohydrate Structures in the β-Carboxyl-Terminal Region of Human Chorionic Gonadotropin," Endocrinology, vol. 122, no. 5, pp. 2054-2063 (1988), the carboxy terminal peptide of hCGβ is identified as an important immunochemical domain that is specific to hCG but which is absent from homologous human luteinizing hormone. Sequence data for the carboxy terminal peptide is also given. Even earlier, in Bidart, J., et al., "Identification of epitopes associated with hCG and the βhCG carboxyl terminus by monoclonal antibodies produced against a synthetic peptide," The Journal of Immunology, vol. 134, no. 1, pp. 457-464 (1985), the authors disclose that they have produced "a library of monoclonal antibodies directed against a 37-amino acid synthetic polypeptide analogous to the carboxyl terminus of hCG." Moreover, the amino acid sequence of this well known synthetic peptide is explicitly provided by Bidart et al. and is therefore not repeated here. Antibodies produced against this synthetic peptide not only recognize and bind with either synthetic or natural carboxy-terminal peptides of hCG, but do not cross-react with other glycoprotein hormones such as LH, TSH and FSH. Disclosure is also provided in this publication as to the location of epitopes on the carboxy terminal peptide, and a variety of monoclonal antibodies which bind with these epitopes. The fact that this publication predates the present specification by almost a decade is a good illustration of the well-known nature of anti-hCGβ mAbs.

More recently, in Krichevsky, A., et al., "Monoclonal antibodies to the native and synthetic βCOOH-terminal portion of human chorionic gonadotropin (hCG) that distinguish between the native and desialylated forms of hCG," Endocrinology, vol.134, no. 3, pp. 1139-1145 (1994), preparation and characterization of anti-hCGβ-CTP mAbs have been described in detail. (Understandably, this publication refers to the "few" CTP mAbs created previously because, relatively speaking, there were overwhelming numbers of mAbs against hCG in existence, generally, compared to the fewer ones specific for βCTP. It should be borne in mind in this context that yet another publication, "A monoclonal antibody against a synthetic peptide is specific for the free native human chorionic gonadotropin β-subunit," Endocrinology, vol. 115, no. 1, pp. 330-336 (1984), identified CTP mAb(s) as early as 1984.) The Krichevsky et al. publication specifically identifies the preparation and characterization of five CTP mAbs, namely, CTP101, CTP102, CTP103, CTP104 and CTP105, the first three of which are the specific exemplary CTP mAbs which were experimentally used in the animal model to establish the ability of CTP mAbs to destroy malignant brain cells in both animals and humans. The same three CTP mAbs have likewise been used in vitro and in vivo to establish the presence of HCGβ-CTP in malignant brain cells in both animals and humans. Independent cell line (hybridoma) deposits of CTP101, CTP102 and CTP103 were made with the American Type Culture Collection (ATCC) on June 13, 1994, and ATCC accession numbers shall be provided as soon as they are received.

The present invention, then, is the treatment of brain tissue by injecting at least one monoclonal antibody against hCGβ-CTP directly into the brain tissue to be treated. Usually the monoclonal antibody will be administered together with at least one pharmaceutically acceptable vehicle compatible with cerebrospinal fluid. The "injection" administration may be literal, by stereotactic injection techniques, or may be figurative in the sense that somehow the mAb(s) are brought into direct contact with the brain tissue. This may be done without stereotactic injection by, for example, administering the mAb(s) intraarterially together with an agent which alters the blood-brain barrier enough to achieve transport of at least some of the mAb(s) into the actual interstices of the brain cells.

A pharmaceutically acceptable vehicle such as sterile physiological saline or sterile physiologically compatible buffer is suitable for use as a carrier for the present mAb(s) as long as it is also compatible with cerebrospinal fluid. Generally, doses between 0.01 and 800 µg/day/10⁶ tumor cells of the mAb(s) are used for injection into human brain tissue, preferably 12-200 µg/day/10⁶ tumor cells. Approved stereotactic injection systems are known in the art.

Examples of administration of CTP mAb(s) in the animal model as predictive of efficacy in humans are provided below. The examples are intended to be illustrative, not limiting.

### Example 1

The C-6 glioma is a malignant astrocytoma initially produced by Benda, P., et al., "Differentiated rat glial cell strain in tissue culture," Science, vol. 161, pp. 370-373 (1968), by injections of N-nitrosomethylurea. This classical tumor model is pathologically similar to the most common malignant glial tumor, glioblastoma multiforme. Recent in vivo and in vitro experiments have shown that both human and C-6 rat glioma cells express hCGβ-CTP, moreover. Correlation between the rat and human model is twofold, therefore: the C-6 glioma was already accepted as a rat model correlating with human glioma, and the fact that both human and C-6 rat glioma cells both express hCGβ-CTP is further evidence of their equivalent susceptibility to treatment with CTP mAb(s).

Two rats were simultaneously inoculated intracerebrally with 1x10⁶ rat C-6 glioma cells. Cells were infused over a 30 minute period via a 28-gauge injector cannula inserted into a previously implanted stainless steel guide cannula. As shown in Figure 3, rat C6-3 showed reductions in body weight and 24 hour spontaneous activity levels (monitored by photo beam interruptions) on the twelfth and thirteenth post-inoculation days respectively. NMR imaging on the seventeenth day revealed the presence of a large intracerebral tumor as shown in Figures 1 and 2. Body weight had dropped 15% by the eighteenth day, at which time intratumoral infusion by osmotic minipump of CTP103 was initiated at a rate of 12 µg/day. Both body weight and activity levels improved during the two week infusion after which the rat was again imaged. Comparison of the pre- and post-treatment images of the brain both in coronal section (Figure 1) and horizontal section (Figure 2) indicate a reduction in tumor volume. The second tumor-inoculated rat showed reductions in body weight beginning on the nineteenth day. This rat received no treatment and died on the twenty-fifth day. In contrast, the CTP103 infused rat currently continues to gain weight and shows normal levels of spontaneous activity at 46 days post-inoculation.

### Example 2

Example 1 is repeated except CTP101 and CTP102 are substituted for CTP103. All three of CTP101, CTP102 and CTP103 are effective to reduce dramatically the size of gliomal tumors of the brain.

### Example 3

Referring once again to Figures 1-3, and in particular to Figure 1, it is helpful to visualize what is represented in these NMR scans as follows. The images are arranged sideways on the page, so that there is a top row of three images and a bottom row of three images. The top row of images shows the brain of rat C6-3 one day before the start of the present treatment. The white areas in the images are the brain tumor. The bottom row of images shows the brain of rat C6-3 fifteen days later. Again, the white areas in the images are the brain tumor. Those with and without formal training in reading NMR images can immediately appreciate that, one day prior to treatment, the tumor was so large it had virtually taken over more than half of the entire brain. Fifteen days later, only a drastically reduced tumor remains. The reduction in tumor volume is so astonishing that, at least in layman's terms, the tumor can be seen almost to have vanished.

Although the invention has been described with respect to specific materials and methods in the foregoing description, these various specifics are in many cases exemplary only and the invention is only to be limited insofar as is set forth in the accompanying claims.

## Claims

1. A method for treating brain neoplasm, comprising the steps of:
identifying the location of suspected neoplasm in the brain, in an animal or patient to be treated; and
effecting a means of delivery of at least one monoclonal antibody to said location of suspected neoplasm, said at least one monoclonal antibody being characterized by its ability specifically to react with an epitope present on the carboxy-terminal peptide of the beta (β) subunit of human chorionic gonadotropin;
wherein said monoclonal antibody lyses the brain neoplasm substantially without affecting normal brain cells of said animal or patient.

2. The method according to claim 1, wherein the step of effecting a means of delivery of monoclonal antibody to said suspected neoplasm is by direct intraarterial administration in order to bypass the blood brain barrier, to provide said at least one monoclonal antibody to said suspected neoplasm.

3. The method according to claim 2, wherein said intraarterial administration is intratumoral administration.

4. The method according to claim 1, wherein the step of effecting a means of delivery of monoclonal antibody to said suspected neoplasm is by stereotactic administration.

5. The method according to claim 1, wherein the step of effecting a means of delivery of monoclonal antibody to said suspected neoplasm is by direct intraarterial administration in conjunction with an osmotic agent to disrupt temporarily the blood-brain barrier to allow at least one monoclonal antibody to reach said suspected neoplasm.

6. The method according to claim 1 wherein said suspected neoplasm is a suspected malignant tumor.

7. The method according to claim 1 wherein said at least one monoclonal antibody is selected from the group consisting of the monoclonal antibodies named CTP101, CTP102, and CTP103.

8. The method according to claim 7 wherein said at least one monoclonal antibody is administered to a human patient in the amount of 0.01-800 µg/day/10⁶ tumor cells.

9. A pharmaceutical composition for treating an animal or patient having suspected brain neoplasm, comprising: at least one monoclonal antibody which specifically reacts with an epitope present on the carboxy-terminal peptide of the beta (β) subunit of human chorionic gonadotropin in admixture with a physiologically compatible and cerebrospinal-fluid-compatible vehicle.

10. The pharmaceutical composition according to claim 9 wherein said at least one monoclonal antibody is selected from the group consisting of monoclonal antibodies named CTP101, CTP102 and CTP103.

11. The pharmaceutical composition according to claim 9 wherein said physiologically compatible and cerebrospinal-fluid-compatible vehicle is selected from the group consisting of sterile physiologically compatible buffer and sterile physiologically compatible saline.
